# EUROPEAN PATENT SPECIFICATION

(11) **EP 1 641 351 B1**
(45) Date of publication and mention of the grant of the patent: **04.06.2014**
(21) Application number: 04756797.9
(22) Date of filing: 02.07.2004
(51) Int. Cl.: A23K 1/16, A23K 1/18, A61K 31/095, A61P 25/28, A23L 1/305

(54) **COMPOSITIONS AND METHODS FOR DECREASING AGE-RELATED DETERIORATION IN MENTAL ACTIVITIES IN COMPANION ANIMALS**
ZUBEREITUNG UND VERFAHREN FÜR DIE VERMINDERUNG DER VERSCHLECHTERUNG DER GEISTLICHEN FÄHIGKEIT IN ALTERNDEN HAUSTIEREN
COMPOSITIONS ET PROCEDES PERMETTANT DE DIMINUER LA DETERIORATION LIEE A L'AGE DES ACTIVITES MENTALES DES ANIMAUX DE COMPAGNIE

(30) Priority: 03.07.2003 US 613604
(43) Date of publication of application: 05.04.2006
(73) Proprietor: Hill's Pet Nutrition, Inc., Topeka, KS 66603 (US)
(72) Inventor: ZICKER, Steven, C., Lawrence, KS 66049 (US); HAYWARD, Larry, H., Topeka, KS 66617 (US); JEWELL, Dennis, E., Perry, KS 66073 (US)
(74) Representative: Jenkins, Peter David
(86) International application number: PCT/US2004/021930
(87) International publication number: WO 2005/006877

(56) References cited:
- WO-A-00/44375
- WO-A-01/17366
- WO-A-02/15719
- WO-A-02/45525
- WO-A-03/015695
- US-A1- 2001 043 983
- DATABASE BIOSIS [Online] BIOSCIENCES INFORMATION SERVICE, PHILADELPHIA, PA, US; Growth Development and Aging 1999; 63: 61-70, 1999 PEACHEY SE, DAWSON JM, HARPER EJ: "The effect of ageing on nutrient digestibility by cats fed tallow-, sunflower oil- or olive oil-enriched diets" Database accession no. PREV199900525302 XP002300922

## Description

### FIELD

The present invention relates to companion-animal diet compositions and, more particularly, to companion-animal diet compositions and methods for decreasing the deterioration in mental activities such as that associated with aging.

### BACKGROUND

Companion animals such as dogs and cats can suffer an age-related decline in mental activities associated with thinking, learning and memory (Houpt, K.A., Cognitive Dysfunction in Geriatric Cats, August J.R. ed, Consultations in Feline Internal Medicine 4th ed., W.B. Saunders, Philadelphia, PA 2001, pp. 583-591). Additionally, behavioral changes and physical changes can be manifested in aging cats in association with the changes in mental capacity. Various possible causes thought to be responsible for this lessening of capacity have been advanced, however effective remedial approaches are not currently available.

WO 02/45525 describes a companion pet diet meeting ordinary nutritional requirements of an aged pet and further comprising a sufficient amount of an antioxidant or mixtures thereof to inhibit the deterioration of the mental capacity of the aged pet.

### SUMMARY

Accordingly, the inventors herein have succeeded in discovering that the presence of one or more sulfur-containing antioxidants, in particular, sulfur-containing amino acids, together with at least one omega-3 fatty acid and an additional antioxidant selected from the group consisting of vitamin E, vitamin C, 1-carnitine and mixtures thereof, in the diet of a cat inhibits, i.e. prevents or reverses deterioration of mental capacity of the cat upon aging. Feeding a cat such a diet composition can bring about significantly increased physical activity of the cat.

Thus, in a first aspect the present invention provides a use of at least one sulfur-containing antioxidant, at least one omega-3 fatty acid and an additional antioxidant selected from the group consisting of vitamin E, vitamin C, 1-carnitine and mixtures thereof, in the manufacture of a diet composition for decreasing deterioration of mental capacity in a feline, wherein decreasing the deterioration of mental capacity comprises altering at least one behavioural attribute in a feline, in a manner indicative of decreased deterioration of mental capacity. In a second aspect the present invention provides a feline diet composition comprising at least one sulfur-containing antioxidant, at least one omega-3 fatty acid and at least two additional antioxidants selected from the group consisting of vitamin E, vitamin C, and 1-carnitine, for use in decreasing deterioration of mental capacity in a feline, wherein decreasing the deterioration of mental capacity comprises altering at least one behavioural attribute in a manner indicative of decreased deterioration of mental capacity, wherein the at least one omega-3 fatty acid is present in the diet composition at a concentration of at least about 0.05 wt. The. Feeding the diet prevents or reverses mental deteriorationin the companion animal.

In various embodiments, the sulfur-containing amino acids or antioxidants in the methods and compositions of the present invention can include one or more sulfurcontaining amino acid selected from the group consisting of cysteine, methionine, taurine, glutathione, s-adenosyl methionine, n-acetyl cysteine, cystathionine, cysteic acid, cysteine sulfinic acid, cystine, methionine sulfone, methionine sulfoxide, betaine, methyl hydroxy analog of methionine and mixtures thereof or a methyl ester of said sulfur-containing amino acids such as methionine methyl ester. In particular, the sulfur-containing amino acid or antioxidant can be methionine at a concentration of, for example, from about 0.8 wt. % to about 2.0 wt; cysteine at a concentration of, for example, from about 0.2 wt. % to about 0.7 wt. % or a mixture of cysteine and methionine at a concentration of from about 1.0 wt. % to about 2.2 wt. %. In various, but not all embodiments, the sulfur-containing antioxidant can be a sulfur-containing antioxidant other than lipoic acid.

In various embodiments, the altered behavioral attribute measured as an indication of decreased deterioration of mental capacity can be decreased wandering aimlessly, decreased inappropriate elimination, decreased excessive vocalization, increased initiating play, decreased sleeping, decreased purring, decreased demanding attention, decreased sitting in lap and/or increased physical activity.

In various embodiments, the omega-3 fatty acid can be docosahexaenoic acid, eicosapentaenoic acid, alpha-linolenic acid or combinations thereof at a concentration of at least about 50 ppm and the additional antioxidant can be vitamin E at a concentration of at least about 500 ppm, vitamin C at a concentration of at least about 50 ppm and 1-carnitine at a concentration of at least about 100 ppm (on a dry matter basis).

The companion cat diet meets ordinary nutritional requirements of the cat of at least one year of age

### DETAILED DESCRIPTION

The diet fed to an aging companion cat, can be a standard normal diet such as that shown in Table 1 for feeding to an adult cat

**Table 1**

| **Component** | **Target** |
|---|---|
| Protein (% of dry matter) | ≥ 30 |
| Fat (% of dry matter) | ≥ 10 |
| Phosphorous (% of dry matter) | ≥ 0.5 |
| Sodium (% of dry matter) | ≥ 0.2 |

Similar diets can be fed to adult cats of greater than about one year of age and older cats of about 7 years of age or greater.

Adding significant quantities of a sulfur-containing antioxidant, in particular, a sulfur-containing amino acid to the companion cat diet can bring about significant and demonstrative changes in the behavior, as specifically shown by increased overall physical activity in an aged cat or by preventing such occurrence of decreased physical activity upon feeding the diet to an adult cat of greater than one year of age. The term, aged is intended to mean, in general, a cat of at least seven years.

The loss of mental capacity with age can be manifested in numerous ways. In a cat, for example, it can be manifested as disorientation, house soiling, altered sleep-wake patterns, decreased interaction with family members and pets, excessive vocalization and diminished physical activity.

By administering the diet of the present invention, an aging cat's physical activity can be enhanced. Although the present diet composition can be used to prevent or reverse age-related deterioration in mental activities, deterioration in mental activities due to causes other than aging such as deterioration due to neurodegenerative diseases can also be prevented or reversed.

A dietary antioxidant, or precursor thereof, can be defined as "a substance in foods that significantly decreases the adverse effects of reactive species, such as reactive oxygen and nitrogen species, on normal physiological function in humans". (Dietary Reference Intakes of Vitamin C, Vitamin E, Selenium, and Carotenoids, Food and Nutrition Board Institute of Medicine, National Academy Press, Washington, D.C., April, 2000, p. 42, said reference being incorporated in its entirety by reference).

Numerous antioxidants can be found in nature and many of such antioxidants are sulfur-containing antioxidants. For example, while not intending to be bound by any theoretical mechanism of action, the sulfur-containing amino acid, methionine, is believed to possess free-radical scavenging activity by virtue of its containing a sulfur which is oxidizable, as well as its having chelating ability. Methionine can also serve as precursor of other antioxidant compounds such as, for example, cysteine. As another example, the sulfur-containing amino acid, cysteine, also contains an oxidizable sulfur and this amino acid can serve as a precursor of the antioxidant glutathione. In a further example of a sulfur-containing antioxidant, the sulfonic amino acid, taurine, is believed to act as an antioxidant by reacting with excess hypochlorite produced in the process of phagocytosis to form N-chlorotaurine. Specific, non-limiting examples of sulfur containing antioxidants include sulfur-containing amino acids such as cysteine, methionine taurine, glutathione, s-adenosyl methionine, n-acetyl cysteine, cystathionine, cysteic acid, cysteine sulfinic acid, cystine, methionine sulfone methionine sulfoxide, betaine, methyl hydroxy analog of methionine, sulfur containing amino acids in addition to those listed above, methyl esters of amino acids such as methionine methyl ester, and the like including other sulfur-containing substance exhibiting the properties described above.

The sulfur-containing antioxidants or sulfur-containing amino acids of the present invention can be naturally occurring or synthetic substances. In various embodiments, the sulfur-containing antioxidants include antioxidants other than lipoic acid.

Other antioxidants are present in the diet compositions of the present invention. Such antioxidants include vitamin E, vitamin C and 1-carnitine. Additionally, omega-3 fatty acids are also present. Vitamin E can be administered as a tocopherol or a mixture of tocopherols and various derivatives thereof such as esters like vitamin E acetate, succinate, palmitate, and the like. The alpha form is preferable but beta, gamma and delta forms can be included. The d form is preferable but racemic mixtures are acceptable. The forms and derivatives will function in a vitamin E like activity after ingestion by the pet. Vitamin C can be administered in this diet as ascorbic acid and its various derivatives thereof such as calcium phosphate salts, cholesteryl salt, 2-monophosphate, and the like which will function in a vitamin C like activity after ingesting by the pet. The above vitamins can be in any form such as liquid, semisolid, solid and heat stable form. L-carnitine can be administered in the diet and various derivatives of carnitine such as the salts such as the hydrochloride, fumarate and succinates, as well as acetylated carnitine, and the like can be used. Omega-3 fatty acids are found in natural sources such as fish oil or vegetable matter. Omega-3 fatty acids found in nature include eicosapentaenoic acid (EPA), docosahexaenoic acid (DHA) and alpha-linolenic acid (ALA).

The diet compositions of the present invention can be in the form of wet cat foods, semi-moist cat foods, dry cat foods and cat treats. Wet cat food generally has a moisture content greater than about 65%. Semi-moist cat food typically has a moisture content between about 20% and about 65% and may include humectants, potassium sorbate, and other ingredients to prevent microbial growth (bacteria and mold). Dry cat food (kibble) generally has a moisture content below about 10% and its processing typically includes extruding, drying and/or baking in heat. Cat treats typically may be semi-moist, chewable treats; dry treats in any number of forms; chewable bones or baked, extruded or stamped treats; confection treats; or other kinds of treats as is known to one skilled in the art.

The quantities administered in a nutritionally balanced diet, all as wt % (dry matter basis) of the diet, are calculated as the active material, *per se,* that is measured as free material. The maximum amounts employed should not bring about toxicity.

Methionine can be present in the diet compositions of the present invention at a concentration of at least about 0.8 wt. %, at least about 0.9% wt. %, at least about 1.0 wt. %, at least about 1.1 wt. % up to about 1.5% or greater. Cysteine can be present in the diet compositions of the present invention at concentrations of at least about 0.2 wt. %, at least about 0.3 wt. %, at least about 0.4 wt. %, at least about 0.5% up to about 0.7% or greater. The combination of methionine and cysteine can also be present at a total concentration of sulfur amino acids of at least about at least about 1.0 wt. %, at least about 1.2 wt. %, at least about 1.4 wt. %, at least about 1.5 wt. %, at least about 1.6 wt. %, up to about 2.2 wt. %.

Vitamin E can be present at a concentration of at least about 100 ppm, at least about 150 ppm, at least about 300 ppm, at least about 500 ppm on a weight basis up to a maximum non-toxic concentration. Maximum concentration of vitamin E can be up to about 1,000 ppm, up to about 1,500 ppm, up to about 2,000 ppm on a weight basis or greater.

Vitamin C can be present at a concentration of at least about 50 ppm, at least about 75 ppm, at least about 100 ppm, at least about 200 ppm up on a weight basis up to a maximum non-toxic concentration. Maximum concentration of vitamin C can be up to about 500 ppm, up to about 600 ppm on a weight basis or greater.

In various embodiments, the diet compositions of the present invention can also contain 1-carnitine at a concentration of at least about 100 ppm, at least about 200 ppm, or at least about 500 ppm on a weight basis up to a maximum non-toxic concentration. Maximum concentration of 1-carnitine can be up to about 4,000 ppm, up to about 5,000 ppm, up to about 6,000 on a weight basis or greater.

Omega-3 fatty acids can be present in the diet compositions at concentrations of at least about 0.1 wt. %, at least about 0.2 wt. %, at least about 0.3 wt. % up to a maximum non-toxic concentration. Typically, one or more omega-3 fatty acids can be present in the diet at a concentration at a total concentration of omega-3 fatty acids of about 0.3 wt. %, about 0.4 wt. % or about 0.5 wt. % or greater.

Other materials can also be present if desired. These materials and their quantities are shown below:
Beta-carotene at about 1-15 ppm can be employed.
Selenium at about 0.1 up to about 5 ppm can be employed.
Lutein at least about 5 ppm can be employed.
Tocotrienols at least about 25 ppm can be employed.
Coenzyme Q10 at least about 25 ppm can be employed.
Soy isoflavones at least about 25 ppm can be used.
Ginkgo Biloba at least 50 ppm of extract or 1% of diet can be used.

### EXAMPLE

This study illustrates the effect of a diet containing increased amounts of methionine and cysteine along with increased amounts of vitamin E, vitamin C, 1-carnitine and the omega-3 fatty acids, docosahexaenoic acid, eicosapentaenoic acid, and alpha-linolenic acid on behavioral characteristics indicative of mental deterioration associated with aging.

Aged cats were enrolled into the trial at 3 different veterinary hospital sites. Older cats of approximately 10 years of age were screened for physical health, clinical bloodwork (to assess liver, thyroid, kidney, endocrine and other health indices), and urinary tract health. Cats were required to have no overt health problems based on the physical and other evaluations. Owners were then asked to assess the behavior of their cat compared to when it was 8 years of age in 22 behavioral attributes.

Forty-six (46) cats fulfilled the health requirements and were enrolled into the study. Cats were assigned randomly to either a test or control food and re-evaluated by questionnaire after 30 days of feeding the assigned food. Both owners and the veterinarian overseeing the trial were masked to the identity of the food assigned. Food compositions were as shown below in Table 2.

**Table 2**

| Concentration of Key Nutrients in Feline Test and Control Formulas in wt % on a dry matter basis | | |
|---|---|---|
| **Analyte** | **Test** | **Control** |
| Protein | 33.5 | 32.8 |
| Fat | 18.1 | 19.8 |
| Calcium | 1.02 | 0.909 |
| Phosphorous | 0.66 | 0.65 |
| Magnesium | 0.069 | 0.067 |
| Taurine | 0.2161 | 0.1946 |
| Vitamin E (IU/kg) | 1178 | 114 |
| Vitamin C (ppm) | 175 | <10 |
| Carnitine (ppm) | 562 | 15 |
| Docosahexaenoic acid | 0.123 | 0.026 |
| Eicosapentaenoic acid | 0.11 | 0.024 |
| alpha-linolenic | 0.279 | 0.119 |
| Methionine | 1.29 | 0.932 |
| Cysteine | 0.61 | 0.475 |
| Met+Cys | 1.902 | 1.407 |

Baseline and 30 day data from 46 enrolled cats was assessed by statistical analysis for between group and within group differences. Baseline data showed no significance between group differences for all questions asked at the start of the study. However, significant differences were present in 9 of the 22 current behavioral assessments asked of owners in comparison to when their cat was 8 years of age (Table 3).

**Table 3**

| Baseline Data Compared to Cats 8 Years of Age | |
|---|---|
| **Question** | **Significant Change** |
| Wandering aimlessly | More |
| Inappropriate elimination | More |
| Excess vocalization | More |
| Initiate play | Less |
| Sleeping | More |
| Purring | More |
| Demand attention | More |
| Sit in lap | More |
| Active | Less |

Upon completion of the 30 day feeding time point of the study, analysis of the results revealed a significant increase in one behavioral assessment, increased activity, in the group receiving test diet compared to the value in the group receiving control diet. This result, coupled with the baseline data, showed that the owners noticed an increased activity similar to that of a younger animal within 30 days of dietary change.

## Claims

1. Use of at least one sulfur-containing antioxidant, at least one omega-3 fatty acid and an additional antioxidant selected from the group consisting of vitamin E, vitamin C, 1-carnitine and mixtures thereof, in the manufacture of a diet composition for decreasing deterioration of mental capacity in a feline, wherein decreasing the deterioration of mental capacity comprises altering at least one behavioural attribute in a feline, in a manner indicative of decreased deterioration of mental capacity.

2. Use according to claim 1, wherein the at least one sulfur-containing antioxidant is at least one sulfur-containing antioxidant other than lipoic acid.

3. Use according to claim 1, wherein the sulfur-containing antioxidant is one or more sulfur-containing amino acid selected from the group consisting of cysteine, methionine, taurine, glutathione, s-adenosyl methionine, n-acetyl cysteine, cystathionine, cysteic acid, cysteine sulfuric acid, cystine, methionine sulfone, methionine sulfoxide, betaine, methyl hydroxy analog of methionine and mixtures thereof or a methyl ester of one or more said sulfur-containing amino acids such as methionine methyl ester.

4. Use according to claim 3, wherein the at least one sulfur-containing antioxidant is methionine, cysteine or a mixture of cysteine and methionine.

5. Use according to claim 4, wherein the methionine is present at a concentration of from about 0. 8 wt. % to about 1.5 wt. %.

6. Use according to claim 4, wherein the cysteine is present in an amount of from about 0.2 wt. % to about 0.7 wt. %.

7. Use according to claim 4, wherein the mixture of cysteine and methionine is present in a total amount of from about 1.0 wt. % to about 2.2 wt. %.

8. Use according to claim 1, wherein the at least one altered behavioural attribute is selected from the group consisting of decreased wandering aimlessly, decreased inappropriate elimination, decreased excessive vocalization, increased initiating play, decreased sleeping, decreased purring, decreased demanding attention, decreased sitting in lap and increased physical activity.

9. Use according to claim 8, wherein the at least one altered behavioural attribute is increased physical activity.

10. Use according to claim 1, wherein the additional antioxidant comprises vitamin E at a concentration of at least about 500 ppm, vitamin C at a concentration of at least about 50 ppm and 1-carnitine at a concentration of at least about 100 ppm.

11. Use according to claim 1, wherein the at least one omega-3 fatty acid is selected from the group consisting of docosahexaenoic acid, eicosapentaenoic acid, alphalinolenic acid and combinations thereof.

12. Use according to claim 1, wherein the at omega-3 fatty acid is present in the diet composition at a concentration of at least about 0.05 ppm.

13. A feline diet composition comprising at least one sulfur-containing antioxidant, at least one omega-3 fatty acid and at least two additional antioxidants selected from the group consisting of vitamin E, vitamin C, and 1-carnitine, for use in decreasing deterioration of mental capacity in a feline, wherein decreasing the deterioration of mental capacity comprises altering at least one behavioural attribute in a manner indicative of decreased deterioration of mental capacity, wherein the at least one omega-3 fatty acid is present in the diet composition at a concentration of at least about 0.05 wt %.

14. A composition according to claim 13, wherein the at least one sulfur-containing me antioxidant is at least one sulfur-containing antioxidant other than lipoic acid.

15. A composition according to claim 13, wherein the sulfur-containing antioxidant is one or more sulfur-containing amino acid selected from the group consisting of cysteine, methionine, taurine, glutathione, s-adenosyl methionine, n-acetyl cysteine, cystathionine, cysteic acid, cysteine sulfinic acid, cystine, methionine sulfone, methionine sulfoxide, betaine, methyl hydroxy analog of methionine and mixtures thereof or a methyl ester of one or more said sulfur-containing amino acids such as methionine methyl ester.

16. A composition according to claim 15, wherein the at least one sulfur-containing antioxidant is methionine, cysteine or a mixture of cysteine and methionine.

17. A composition according to claim 16, wherein the methionine is present at a concentration of from about 0.8 wt. % to about 2.0 wt. %.

18. A composition according to claim 16, wherein the cysteine is present in an amount of from about 0.2 wt. % to about 0.7 wt. %.

19. A composition according to claim 16, wherein the mixture of cysteine and methionine is present in a total amount of from about 1.0 wt. % to about 2.2 wt. %.

20. A composition according to claim 13, wherein the at least one altered behavioural attribute is selected from the group consisting of decreased wandering aimlessly, decreased inappropriate elimination, decreased excessive vocalization, increased initiating play, decreased sleeping, decreased purring, decreased demanding attention, decreased sitting in lap and increased physical activity.

21. A composition according to claim 20, wherein the at least one altered behavioural attribute is increased physical activity.

22. A composition according to claim 13, wherein the at least two additional antioxidant comprises vitamin E at a concentration of at least about 500 ppm, vitamin C at a concentration of at least about 50 ppm and 1-carnitine at a concentration of at least about 100 ppm.

23. A composition according to claim 13, wherein the at least one omega-3 fatty acid is selected from the group consisting of docosahexaenoic acid, eicosapentaenoic acid, alphalinolenic acid and combinations thereof.

24. A composition according to claim 13, wherein the omega-3 fatty acid is present in the diet composition at a concentration of at least about 0.1 wt %.

## Patentansprüche

1. Verwendung von mindestens einem schwefelhaltigen Antioxidans, mindestens einer Omega-3-Fettsäure und einem zusätzlichen Antioxidans ausgewählt aus der Gruppe bestehend aus Vitamin E, Vitamin C, 1-Carnitin und Mischungen davon, bei der Herstellung einer Diätzusammensetzung zur Verminderung der Verschlechterung der geistigen Fähigkeiten bei einer Katze, wobei die Verminderung der Verschlechterung der geistigen Fähigkeiten die Veränderung von mindestens einem Verhaltensmerkmal bei einer Katze auf eine Weise einschließt, die eine verminderte Verschlechterung der geistigen Fähigkeiten belegt.

2. Verwendung nach Anspruch 1, wobei das mindestens eine schwefelhaltige Antioxidans mindestens ein von Liponsäure verschiedenes schwefelhaltiges Antioxidans ist.

3. Verwendung nach Anspruch 1, wobei das schwefelhaltige Antioxidans eine oder mehrere schwefelhaltige Aminosäuren ausgewählt aus der Gruppe bestehend aus Cystein, Methionin, Taurin, Glutathion, S-Adenosylmethionin, N-AcetylCystein, Cystathionin, Cysteinsäure, Cysteinschwefelsäure, Cystin, Methioninsulfon, Methioninsulfoxid, Betain, Methylhydroxyanalogon von Methionin und Mischungen davon oder ein Methylester von einer oder mehreren der schwefelhaltigen Aminosäuren, wie Methionin-Methylester, ist.

4. Verwendung nach Anspruch 3, wobei das mindestens eine schwefelhaltige Antioxidans Methionin, Cystein oder eine Mischung von Cystein mit Methionin ist.

5. Verwendung nach Anspruch 4, wobei das Methionin in einer Konzentration von etwa 0,8 Gew.-% bis etwa 1,5 Gew.-% vorhanden ist.

6. Verwendung nach Anspruch 4, wobei das Cystein in einer Konzentration von etwa 0,2 Gew.-% bis etwa 0,7 Gew.-% vorhanden ist.

7. Verwendung nach Anspruch 4, wobei die Mischung von Cystein mit Methionin in einer Gesamtmenge von etwa 1,0 Gew.-% bis etwa 2,2 Gew.-% vorhanden ist.

8. Verwendung nach Anspruch 1, wobei das mindestens eine veränderte Verhaltensmerkmal ausgewählt ist aus der Gruppe bestehend aus vermindertem ziellosen Umherlaufen, vermindertem unangemessenen Ausscheiden, verminderter übermäßiger Lautgebung, erhöhter Verspieltheit, verminderter Schläfrigkeit, vermindertem Schnurren, vermindertem Bedarf an Aufmerksamkeit, vermindertem Schoßsitzen und erhöhter physischer Aktivität.

9. Verwendung nach Anspruch 8, wobei das mindestens eine veränderte Verhaltensmerkmal erhöhte physische Aktivität ist.

10. Verwendung nach Anspruch 1, wobei das zusätzliche Antioxidans Vitamin E in einer Konzentration von mindestens etwa 500 ppm, Vitamin C in einer Konzentration von mindestens etwa 50 ppm und 1-Carnitin in einer Konzentration von mindestens etwa 100 ppm umfasst.

11. Verwendung nach Anspruch 1, wobei die mindestens eine Omega-3-Fettsäure ausgewählt ist bestehend aus der Gruppe bestehend aus Docosahexaensäure, Eicosapentaensäure, Alpha-Linolensäure und Kombinationen davon.

12. Verwendung nach Anspruch 1, wobei die Omega-3-Fettsäure in der Diätzusammensetzung in einer Konzentration von mindestens etwa 0,05 ppm vorhanden ist.

13. Katzendiätzusammensetzung, die mindestens ein schwefelhaltiges Antioxidans, mindestens eine Omega-3-Fettsäure und mindestens zwei zusätzliche Antioxidantien ausgewählt aus der Gruppe bestehend aus Vitamin E, Vitamin C und 1-Carnitin umfasst, zur Verwendung bei der Verminderung der Verschlechterung der geistigen Fähigkeiten einer Katze, wobei die Verminderung der Verschlechterung der geistigen Fähigkeiten die Veränderung von mindestens einem Verhaltensmerkmal bei einer Katze auf eine Weise einschließt, die eine verminderte Verschlechterung der geistigen Fähigkeiten belegt und wobei die mindestens eine Omega-3-Fettsäure in der Diätzusammensetzung in einer Konzentration von mindestens etwa 0,05 Gew.-% vorhanden ist.

14. Zusammensetzung nach Anspruch 13, wobei das mindestens eine schwefelhaltige Antioxidans mindestens ein von Liponsäure verschiedenes schwefelhaltiges Antioxidans ist.

15. Zusammensetzung nach Anspruch 13, wobei das schwefelhaltige Antioxidans eine oder mehrere schwefelhaltige Aminosäuren ausgewählt aus der Gruppe bestehend aus Cystein, Methionin, Taurin, Glutathion, S-Adenosylmethionin, N-Acetylcystein, Cystathionin, Cysteinsäure, Cysteinschwefelsäure, Cystin, Methioninsulfon, Methioninsulfoxid, Betain, Methylhydroxyanalogon von Methionin und Mischungen davon oder ein Methylester von einer oder mehreren der schwefelhaltigen Aminosäuren, wie Methionin-Methylester, ist.

16. Zusammensetzung nach Anspruch 15, wobei das mindestens eine schwefelhaltige Antioxidans Methionin, Cystein oder eine Mischung von Cystein mit Methionin ist.

17. Zusammensetzung nach Anspruch 16, wobei das Methionin in einer Konzentration von etwa 0,8 Gew.-% bis etwa 2,0 Gew.-% vorhanden ist.

18. Zusammensetzung nach Anspruch 16, wobei das Cystein in einer Konzentration von etwa 0,2 Gew.-% bis etwa 0,7 Gew.-% vorhanden ist.

19. Zusammensetzung nach Anspruch 16, wobei die Mischung von Cystein mit Methionin in einer Gesamtmenge von etwa 1,0 Gew.-% bis etwa 2,2 Gew.-% vorhanden ist.

20. Zusammensetzung nach Anspruch 13, wobei das mindestens eine veränderte Verhaltensmerkmal ausgewählt ist aus der Gruppe bestehend aus vermindertem ziellosen Umherlaufen, vermindertem unangemessenen Ausscheiden, verminderter übermäßiger Lautgebung, erhöhter Verspieltheit, verminderter Schläfrigkeit, vermindertem Schnurren, vermindertem Bedarf an Aufmerksamkeit, vermindertem Schoßsitzen und erhöhter physischer Aktivität.

21. Zusammensetzung nach Anspruch 20, wobei das mindestens eine veränderte Verhaltensmerkmal erhöhte physische Aktivität ist.

22. Zusammensetzung nach Anspruch 13, wobei das zusätzliche Antioxidans Vitamin E in einer Konzentration von mindestens etwa 500 ppm, Vitamin C in einer Konzentration von mindestens etwa 50 ppm und 1-Carnitin in einer Konzentration von mindestens etwa 100 ppm umfasst.

23. Zusammensetzung nach Anspruch 13, wobei die mindestens eine Omega-3-Fettsäure ausgewählt ist bestehend aus der Gruppe bestehend aus Docosahexaensäure, Eicosapentaensäure, Alpha-Linolensäure und Kombinationen davon.

24. Zusammensetzung nach Anspruch 13, wobei die Omega-3-Fettsäure in der Diätzusammensetzung in einer Konzentration von mindestens etwa 0,1 Gew.-% vorhanden ist.

## Revendications

1. Utilisation d'au moins un antioxydant contenant du soufre, d'au moins un acide gras oméga-3 et d'un antioxydant supplémentaire sélectionné dans le groupe comprenant la vitamine E, la vitamine C, la 1-carnitine et des mélanges de ceux-ci, dans la fabrication d'une composition alimentaire destinée à réduire la détérioration de la capacité mentale chez un félin, où la réduction de la détérioration de la capacité mentale consiste à altérer au moins un attribut comportemental chez un félin, d'une manière indiquant une détérioration de la capacité mentale réduite.

2. Utilisation selon la revendication 1, dans laquelle le au moins un antioxydant contenant du soufre est au moins un antioxydant contenant du soufre autre que l'acide lipoïque.

3. Utilisation selon la revendication 1, dans laquelle l'antioxydant contenant du soufre est un ou plusieurs acides aminés contenant du soufre sélectionnés dans le groupe comprenant la cystéine, la méthionine, la taurine, le glutathion, la sadénosylméthionine, la n-acétylcystéine, la cystathionine, l'acide cystéique, l'acide cystéine-sulfinique, la cystine, la méthionine sulfone, le sulfoxyde de méthionine, la bétaïne, un analogue méthyl-hydroxy de la méthionine et des mélanges de ceux-ci, ou un ester méthylique d'un ou plusieurs desdits acides aminés contenant du soufre comme l'ester méthylique de la méthionine.

4. Utilisation selon la revendication 3, dans laquelle le au moins un antioxydant contenant du soufre est la méthionine, la cystéine ou un mélange de cystéine et de méthionine.

5. Utilisation selon la revendication 4, dans laquelle la méthionine est présente à une concentration d'environ 0,8 % en poids à environ 1,5 % en poids.

6. Utilisation selon la revendication 4, dans laquelle la cystéine est présente en une quantité d'environ 0,2 % en poids à environ 0,7 % en poids.

7. Utilisation selon la revendication 4, dans laquelle le mélange de cystéine et de méthionine est présent en une quantité totale d'environ 1,0 % en poids à environ 2,2 % en poids.

8. Utilisation selon la revendication 1, dans laquelle le au moins un attribut comportemental altéré est sélectionné dans le groupe comprenant une errance sans but précis réduite, une élimination inappropriée réduite, une vocalisation excessive réduite, une initiation du jeu accrue, un sommeil réduit, un ronronnement réduit, une demande d'attention réduite, une assise sur les genoux réduite et une activité physique accrue.

9. Utilisation selon la revendication 8, dans laquelle le au moins un attribut comportemental altéré est une activité physique accrue.

10. Utilisation selon la revendication 1, dans laquelle l'antioxydant supplémentaire comprend de la vitamine E à une concentration d'au moins environ 500 ppm, de la vitamine C à une concentration d'au moins environ 50 ppm et de la 1-carnitine à une concentration d'au moins environ 100 ppm.

11. Utilisation selon la revendication 1, dans laquelle le au moins un acide gras oméga-3 est sélectionné dans le groupe comprenant l'acide docosahexaénoïque, l'acide éicosapentaénoïque, l'acide alpha-linolénique et des combinaisons de ceux-ci.

12. Utilisation selon la revendication 1, dans laquelle l'acide gras oméga-3 est présent dans la composition alimentaire à une concentration d'au moins environ 0,05 ppm.

13. Composition alimentaire féline comprenant au moins un antioxydant contenant du soufre, au moins un acide gras oméga-3 et au moins deux antioxydants supplémentaires sélectionnés dans le groupe comprenant la vitamine E, la vitamine C, et la 1-carnitine, destinée à être utilisée pour réduire la détérioration de la capacité mentale chez un félin, où la réduction de la détérioration de la capacité mentale consiste à altérer au moins un attribut comportemental d'une manière indiquant une détérioration de la capacité mentale réduite, où le au moins un acide gras oméga-3 est présent dans la composition alimentaire à une concentration d'au moins environ 0,05 % en poids.

14. Composition selon la revendication 13, dans laquelle le au moins un antioxydant contenant du soufre est au moins un antioxydant contenant du soufre autre que l'acide lipoïque.

15. Composition selon la revendication 13, dans laquelle l'antioxydant contenant du soufre est un ou plusieurs acides aminés contenant du soufre sélectionnés dans le groupe comprenant la cystéine, la méthionine, la taurine, le glutathion, la s-adénosylméthionine, la n-acétylcystéine, la cystathionine, l'acide cystéique, l'acide cystéine-sulfinique, la cystine, la méthionine sulfone, le sulfoxyde de méthionine, la bétaïne, un analogue méthyl-hydroxy de la méthionine et des mélanges de ceux-ci, ou un ester méthylique d'un ou plusieurs desdits acides aminés contenant du soufre comme l'ester méthylique de la méthionine.

16. Composition selon la revendication 15, dans laquelle le au moins un antioxydant contenant du soufre est la méthionine, la cystéine ou un mélange de cystéine et de méthionine.

17. Composition selon la revendication 16, dans laquelle la méthionine est présente à une concentration d'environ 0,8 % en poids à environ 2,0 % en poids.

18. Composition selon la revendication 16, dans laquelle la cystéine est présente en une quantité d'environ 0,2 % en poids à environ 0,7 % en poids.

19. Composition selon la revendication 16, dans laquelle le mélange de cystéine et de méthionine est présent en une quantité totale d'environ 1,0 % en poids à environ 2,2 % en poids.

20. Composition selon la revendication 13, dans laquelle le au moins un attribut comportemental altéré est sélectionné dans le groupe comprenant une errance sans but précis réduite, une élimination inappropriée réduite, une vocalisation excessive réduite, une initiation du jeu accrue, un sommeil réduit, un ronronnement réduit, une demande d'attention réduite, une assise sur les genoux réduite et une activité physique accrue.

21. Composition selon la revendication 20, dans laquelle le au moins un attribut comportemental altéré est une activité physique accrue.

22. Composition selon la revendication 13, dans laquelle les au moins deux antioxydants supplémentaires comprennent de la vitamine E à une concentration d'au moins environ 500 ppm, de la vitamine C à une concentration d'au moins environ 50 ppm et de la 1-carnitine à une concentration d'au moins environ 100 ppm.

23. Composition selon la revendication 13, dans laquelle le au moins un acide gras oméga-3 est sélectionné dans le groupe comprenant l'acide docosahexaénoïque, l'acide éicosapentaénoïque, l'acide alpha-linolénique et des combinaisons de ceux-ci.

24. Composition selon la revendication 13, dans laquelle l'acide gras oméga-3 est présent dans la composition alimentaire à une concentration d'au moins environ 0,1 % en poids.
